# EUROPÄISCHE PATENTSCHRIFT

(11) **EP 2 476 388 B1**
(45) Veröffentlichungstag und Bekanntmachung des Hinweises auf die Patenterteilung: **29.10.2014**
(21) Anmeldenummer: 11151143.2
(22) Anmeldetag: 17.01.2011
(51) Int. Cl.: A61B 17/80

(54) **Osteosyntheseplatte, insbesondere Radiusplatte oder Ulnarplatte, zur Stabilisierung von Knochenfrakturen**
Osteosynthesis plate, in particular radius plate or ulnar plate, for stabilising bone fractures
Plaque d'ostéosynthèse, notamment plaque radiale ou plaque Ulnar, destinée à la stabilisation de fractures d'os

(43) Veröffentlichungstag der Anmeldung: 18.07.2012
(73) Patentinhaber: HG Medical GmbH, 82399 Raisting (DE)
(72) Erfinder: Hagenmeyer, Johannes, 86911 Dießen (DE)
(74) Vertreter: Rupprecht, Kay

(56) Entgegenhaltungen:
- US-A1- 2004 102 775
- US-A1- 2005 070 904
- US-A1- 2006 264 949
- US-A1- 2007 123 880

## Beschreibung

Die Erfindung betrifft eine Osteosyntheseplatte, insbesondere Radiusplatte oder UInarplatte, zur Stabilisierung von Knochenfrakturen gemäß dem Oberbegriff des Patentanspruchs 1. Eine derartige Osteosyntheseplatte ist beispielsweise aus US 2007/123880 A1 bekannt.

Eine weitere Osteosyntheseplatte ist aus der WO 02/096309 A1 bekannt. Diese bekannte Osteosyntheseplatte bzw. Knochenplatte umfasst einen stielförmigen Teil und einen löffelartigen Teil. Der löffelartige Teil ist breiter als der stielförmige Teil. In beiden Teilen sind Bohrungen angeordnet, die zur Aufnahme von Knochenschrauben fungieren. Insbesondere weist der stielförmige Teil eine Mehrzahl von Bohrungen auf, die in einer Reihe entlang der Längsachse des stielförmigen Teils angeordnet sind. Jeweils zwei Bohrungen mit unterschiedlichem Bohrungsdurchmesser sind ineinander verschränkt angeordnet, so dass sich entlang des stielförmigen Teils Öffnungen erstrecken, deren Außenkontur der Form einer Acht ähnlich ist.

Im Allgemeinen werden Osteosyntheseplatten zur Fixierung von Knochenbrüchen bzw. Knochenfrakturen bei Röhrenknochen eingesetzt. Dabei wird der löffelartige Teil bzw. Kopfteil der Osteosyntheseplatte im Bereich des Knochenendstücks bzw. der Epiphyse angeordnet und mit Schrauben fixiert. Der stielförmige Teil bzw. Schaftteil wird hingegen mit dem Knochenschaft bzw. der Diaphyse verschraubt. Die Befestigung der Osteosyntheseplatte an dem Röhrenknochen erfolgt derart, dass die getrennten Knochenfragmente in möglichst natürlicher Relativposition zueinander angeordnet sind. Auf diese Weise wird erreicht, dass der frakturierte Knochen in seiner ursprünglichen Form zusammenwächst. Dazu ist es zweckmäßig, dass sich die Osteosyntheseplatte gut an die anatomischen Verhältnisse am betroffenen Knochen anpasst.

Bei der bekannten Osteosyntheseplatte besteht der Nachteil darin, dass durch die geradlinige, insbesondere fluchtende, Anordnung der Bohrungen im stielförmigen Teil eine Ausrichtung der Knochenplatte in Querrichtung erschwert ist. Insbesondere bei Frakturen von Knochen, deren Knochenschaft gegenüber dem Knochenendstück schräg angeordnet ist, wie es beispielsweise bei Elle (Ulna) und Speiche (Radius) der Fall ist, stößt die bekannte Knochenplatte an Grenzen.

Der Erfindung liegt die Aufgabe zugrunde, eine Osteosyntheseplatte, insbesondere Radiusplatte oder Ulnarplatte, zur Stabilisierung von Knochenfrakturen anzugeben, die einfach und schnell an unterschiedliche anatomische Gegebenheiten anpassbar ist. Insbesondere soll eine Osteosyntheseplatte bereitgestellt werden, die während einer Operation, also während des Einsetzens der Osteosyntheseplatte, einfach an die Form des betroffenen Knochens anpassbar ist.

Erfindungsgemäß wird diese Aufgabe durch den Gegenstand des Patentanspruchs 1 gelöst. Bevorzugte Weiterbildungen sind in den Unteransprüchen angegeben.

Die Erfindung beruht auf dem Gedanken, eine Osteosyntheseplatte, insbesondere Radiusplatte oder Ulnarplatte, zur Stabilisierung von Knochenfrakturen anzugeben, die einen Kopfteil zur Anordnung an einem Knochenstück und einen Schaftteil zur Anordnung an einem Knochenschaft umfasst. Der Kopfteil und der Schaftteil weisen jeweils Bohrungen zur Aufnahme von Knochenschrauben auf. Ferner weist der Schaftteil wenigstens ein Langloch zur Aufnahme einer Knochenschraube auf, das zumindest abschnittsweise schräg zur Plattenlängsrichtung, insbesondere zur Schaftteillängsrichtung, angeordnet ist.

Der Vorteil der Erfindung besteht darin, dass durch das schräg angeordnete Langloch eine Ausrichtung der Osteosyntheseplatte nicht nur in Längsrichtung des Knochenschafts, sondern auch quer zum Knochenschaft ermöglicht ist. Dieser Vorteil kommt insbesondere bei der Stabilisierung von Knochenfrakturen des distalen Radius, also dem handgelenknahen Bereich der Speiche, oder der distalen Ulna, also dem handgelenknahen Bereich der Elle, zum Tragen. Gerade der Radius und die Ulna weisen jeweils einen Knochenschaft auf, der zum Knochenendstück schräg bzw. geneigt angeordnet ist. Der Neigungsgrad ist unterschiedlich, insbesondere personenabhängig unterschiedlich. Durch das schräg zur Plattenlängsrichtung, insbesondere zur Schaftteillängsrichtung, angeordnete Langloch kann die erfindungsgemäße Osteosyntheseplatte einfach während der Operation an die anatomische Gegebenheit des zu stabilisierenden Knochens angepasst werden. Die Ausrichtung der zu fixierenden Knochenfragmente wird somit verbessert. Insgesamt ist die erfindungsgemäße Osteosyntheseplatte einfach handhabbar und erleichtert bzw. beschleunigt den Operationsvorgang.

Das Langloch weist einen ersten Schenkel und einen zweiten Schenkel auf, wobei der erste Schenkel im Wesentlichen quer, insbesondere rechtwinklig, zur Plattenlängsrichtung, insbesondere zur Längsrichtung des Schaftteils angeordnet ist. Der zweite Schenkel ist im Wesentlichen schräg, insbesondere geneigt zur Plattenlängsrichtung, insbesondere zur Längsrichtung des Schaftteils, angeordnet. Auf diese Weise wird, einerseits eine Ausrichtung der Osteosyntheseplatte ausschließlich in Querrichtung bzw. zur Seitenachse des betroffenen Knochens ermöglicht, indem die entsprechende Knochenschraube in den ersten Schenkel des Langlochs eingesetzt wird. Alternativ kann die Knochenschraube oder eine weitere Knochenschraube in den schräg verlaufenden zweiten Schenkel eingebracht werden, wobei durch die schräge Anordnung des zweiten Schenkels eine Anpassung der Osteosyntheseplatte nicht nur in Längsrichtung bzw. entlang der Längsachse, sondern auch in Querrichtung, also entlang der Seitenachse, ermöglicht ist.

Des Weiteren ist in einem proximalen Abschnitt des Schaftteils ein weiteres Langloch angeordnet, welches sich geradlinig in Plattenlängsrichtung erstreckt.

Eine weitere Verbesserung in der Handhabbarkeit und Einstellbarkeit der erfindungsgemäßen Osteosyntheseplatte wird gemäß einer bevorzugten Ausführungsform dadurch erreicht, dass das Langloch hakenförmig, insbesondere in Form einer Sieben ausgebildet ist. Das Langloch kann konkret eine Form aufweisen, die der Form der arabischen Zahl Sieben entspricht.

Um den anatomischen Gegebenheiten eines Röhrenknochens gerecht zu werden und einen höheren Grad der Verstellbarkeit in Längsrichtung des Knochens gegenüber einem relativ kürzeren Verstellweg in Querrichtung des Knochens, einzustellen, ist gemäß einer weiteren bevorzugten Ausführungsform vorgesehen, dass der erste Schenkel kürzer als der zweite Schenkel ist. Der rechtwinklig in Plattenlängsrichtung angeordnete erste Schenkel weist also eine Länge auf, die kleiner als die Länge des schräg zur Plattenlängsrichtung verlaufenden zweiten Schenkels ist. Der zweite Schenkel kann überdies einen gekrümmten Verlauf aufweisen. Dies ermöglicht eine besonders präzise Anpassung der Osteosyntheseplatte an den zu stabilisierenden Knochen. Gemäß einem weiteren bevorzugten Ausführungsbeispiel ist vorgesehen, dass der Schaftteil zwei Bohrungen zur Aufnahme von Knochenschrauben aufweist, deren Verbindungslinie schräg zur Plattenlängsrichtung, insbesondere zur Schaftteillängsrichtung, verläuft. Die Verbindungslinie kreuzt das Langloch, insbesondere den zweiten Schenkel des Langlochs. Durch die beiden Bohrungen, deren Verbindungslinie sich mit dem Langloch kreuzt, ist ermöglicht, die Osteosyntheseplatte einfach zu fixieren, nachdem die Position der Osteosyntheseplatte mit Hilfe des Langlochs eingestellt ist.

Das Langloch weist vorzugsweise eine Senkung auf, so dass der Kopf einer Knochenschraube bündig in die Osteosyntheseplatte versenkt werden kann. Damit werden überstehende Kanten im Bereich der Osteosyntheseplatte vermieden, wodurch die Anpassung der Osteosyntheseplatte an das umliegende Gewebe verbessert ist.

Bei einer bevorzugten Ausführungsform der erfindungsgemäßen Osteosyntheseplatte ist zwischen dem Kopfteil und dem Schaftteil eine Kröpfung angeordnet. Die Form der Kröpfung bildet vorzugsweise die Form des Übergangs zwischen der Epiphyse und der Diaphyse des zu behandelnden Knochens nach, so dass die erfindungsgemäße Osteosyntheseplatte besonders gut an den Knochen anpassbar ist.

Der Schaftteil kann bezogen auf die Plattenlängsrichtung eine Länge und eine Breite aufweisen, wobei die Länge größer als die Breite ist. Auf diese Weise wird eine größere Fläche zur Anbringung von Bohrungen bereitgestellt, so dass eine ausreichende Fixierung des Knochens erreicht werden kann. Insbesondere erstreckt sich der Schaftteil im Gebrauch über den Knochenschaft eines zu behandelnden Knochens, wobei durch die Länge des Schaftteils, die größer als die Breite des Schaftteils ist, eine größere Knochenschaftfläche überdeckt und somit eine stärkere Verbindung zwischen dem Schaftteil und dem Knochenschaft erreicht wird.

Der Kopfteil kann bezogen auf die Plattenlängsrichtung eine Breite aufweisen, die größer als die Breite des Schaftteils ist. Auf diese Weise können im Kopfteil eine ausreichende Anzahl von Bohrungen bereitgestellt werden, die den Kopfteil an der Epiphyse des zu behandelnden Knochens befestigen. Im Allgemeinen sind der Kopfteil an die Form der Epiphyse und der Schaftteil an die Form des Knochenschafts angepasst.

Die Erfindung wird im Folgenden anhand eines Ausführungsbeispiels unter Bezugnahme auf die beigefügten, schematischen Zeichnungen näher erläutert. Darin zeigen:
- Figur 1:: eine Draufsicht auf die erfindungsgemäße Osteosyntheseplatte gemäß einem bevorzugten Ausführungsbeispiel;
- Figur 2:: eine Seitenansicht der Osteosyntheseplatte gemäß Figur 1;
- Figur 3:: eine Detailansicht der Osteosyntheseplatte gemäß Figur 1 gemäß Detail A; und
- Figur 4:: eine Querschnittsansicht der Osteosyntheseplatte gemäß Figur 1 entlang der Schnittlinie B-B, dargestellt in Figur 3.

Die in den Figuren dargestellte Osteosyntheseplatte weist einen Kopfteil 10 und einen Schaftteil 20 auf. Der Schaftteil 20 ist insgesamt länglich ausgebildet. Das bedeutet, dass der Schaftteil 20 eine Länge aufweist, die größer als die Breite des Schaftteils 20 ist. Der Kopfteil 10 weist eine Breite auf, die breiter als die Breite des Schaftteils 20 ist. Der Übergang vom relativ schmaleren Schaftteil 20 zum relativ breiteren Kopfteil 10 erfolgt kontinuierlich. Mit anderen Worten weist die Osteosyntheseplatte ausgehend vom Schaftteil 20 eine kontinuierliche, insbesondere kontinuierlich wachsende, Verbreiterung auf, die in den Kopfteil 10 übergeht.

Im Übergang vom Schaftteil 20 zum Kopfteil 10 ist ein im Wesentlichen dreieckförmiger Durchbruch 11 angeordnet. Die Seitenflächen des im Wesentlichen dreieckförmigen Durchbruchs 11 sind dabei in das Innere des Durchbruchs 11 vorgewölbt. Insbesondere sind im Übergangsbereich zwischen dem Schaftteil 20 und dem Kopfteil 10 Bereiche, die nur wenig oder überhaupt keine Kräfte aufnehmen, ausgespart, um das Gewicht der Osteosyntheseplatte zu reduzieren. Der Durchbruch 11 trägt also Wesentlich zur Materialeinsparung bei.

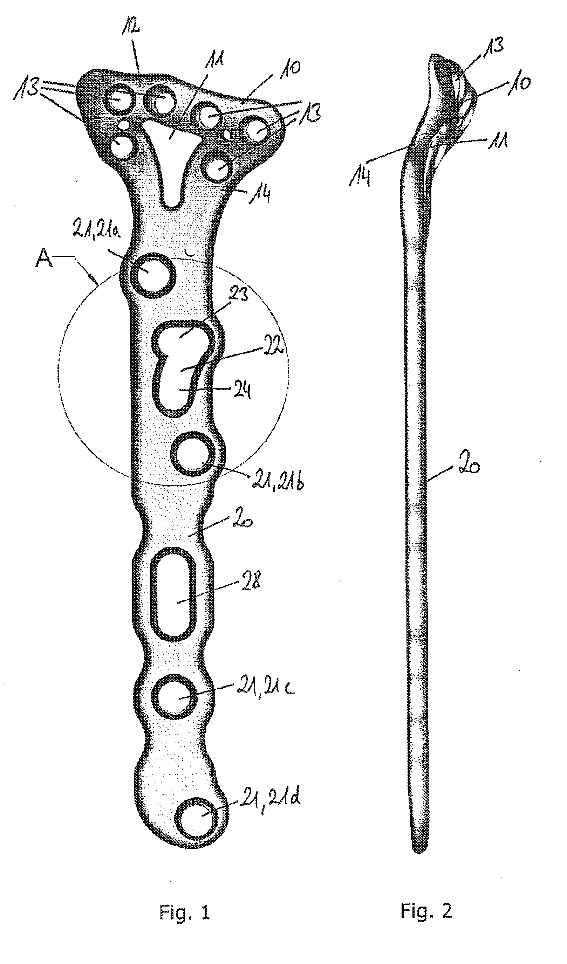

Insgesamt ist die Osteosyntheseplatte Y-förmig ausgebildet, wobei im Bereich des Übergangs vom Schafteil 20 zum Kopfteil 10 eine sich aufspreizende Gabelform ausgebildet ist. Die Spitzen der Y-förmigen Gabel sind durch einen quer verlaufenden Steg 12 des Kopfteils verbunden. Im Steg 12 sowie in der Y-förmigen Gabelung des Übergangs zwischen dem Schaftteil 20 und dem Kopfteil 10 sind Bohrungen zur Aufnahme von Knochenschrauben angeordnet. Insbesondere sind im Steg 12 vier Bohrungen 13 angeordnet. Zwei weitere Bohrungen 13 sind im Übergang zwischen Schaffteil 20 und Kopfteil 10 angeordnet. Insbesondere sind die weiteren Bohrungen 13 in einer Kröpfung 14 angeordnet, die sich vom Schafteil 20 zum Kopfteil 10 erstreckt. Die Kröpfung 14 ist besonders gut in Figur 2 erkennbar. Insbesondere bildet die Kröpfung 14 einen S-förmigen Übergang vom Kopfteil 10 zum Schaftteil 20.

Der Schaftteil 20 umfasst ebenfalls mehrere Bohrungen 21, die zur Aufnahme von Knochenschrauben vorgesehen sind. Die Bohrungen 21 sind über den Schaftteil 20 versetzt, insbesondere bezogen auf die Plattenlängsrichtung versetzt, angeordnet. Wenigstens zwei Bohrungen 21 im Schaftteil 20 weisen eine Verbindungslinie auf, die die Bohrungsmittelpunkte bzw. Bohrungsachsen der beiden Bohrungen 21 im Schaftteil 20 verbindet und schräg zur Plattenlängsrichtung, insbesondere zur Schaftteillängsrichtung, verläuft. Vorzugsweise sind ausgehend vom Kopfteil 10 eine erste Bohrung 21a und eine zweite Bohrung 21b derart angeordnet, dass die Verbindungslinie zwischen der ersten Bohrung 21a und der zweiten Bohrung 21b schräg zur Plattenlängsrichtung verläuft. Insbesondere ist die erste Bohrung 21a an einem distalen Ende des Schaftteils 20 angeordnet. Die in proximale Richtung des Schaftteils 20 nachfolgende Bohrung 21 wird durch die zweite Bohrung 21b gebildet.

In diesem Zusammenhang wird darauf hingewiesen, dass sich die Richtungsangaben "distal" und "proximal" auf die Anordnung der Osteosyntheseplatte im Gebrauch beziehen. Dabei wird die Osteosyntheseplatte derart an einem Knochen angeordnet, dass der Kopfteil 10 an einem distalen Ende des Knochens angeordnet ist und sich der Schaftteil 20 in proximale Richtung erstreckt. Dabei sind distal angeordnete Bereiche weiter von der Körpermitte des Patienten entfernt angeordnet als proximal angeordnete Körperbereiche.

Zwischen der ersten Bohrung 21a und der zweiten Bohrung 21b ist ein Langloch 22 zur Aufnahme einer Knochenschraube angeordnet. Das Langloch 22 ist zumindest abschnittsweise schräg zur Plattenlängsrichtung ausgerichtet. Mit anderen Worten weist das Langloch 22 wenigstens einen Abschnitt auf, dessen Mittelachse eine Plattenlängsachse unter einem Winkel schneidet. Das Langloch 22 weist vorzugsweise eine hakenförmige Kontur auf. Bei dem gezeigten Ausführungsbeispiel ist das Langloch im Wesentlichen in Form einer Sieben ausgebildet. Die Außenkontur des Langlochs 22 entspricht insbesondere der Form der arabischen Zahl Sieben.

Konkret weist das Langloch 22 einen ersten Schenkel 23 und einen zweiten Schenkel 24 auf. Der erste Schenkel 23 erstreckt sich im Wesentlichen rechtwinklig zur Plattenlängsachse bzw. Plattenlängsrichtung. Der zweite Schenkel 24 verläuft hingegen schräg zur Plattenlängsrichtung. Der zweite Schenkel 24 ist bezogen auf die Plattenlängsrichtung bzw. Plattenlängsachse geneigt angeordnet. Insbesondere ist der zweite Schenkel 24 derart schräg zur Plattenlängsrichtung angeordnet, dass zwischen dem ersten Schenkel 23 und dem zweiten Schenkel 24 ein spitzer Winkel gebildet ist.

Der erste Schenkel 23 und der zweite Schenkel 24 bilden gemeinsam das Langloch 22.

Die schmalen Seiten des ersten Schenkels 23 und des zweiten Schenkels 24 sind jeweils durch Teilkreise geschlossen, wobei die Teilkreise des Langlochs 22 einen einheitlich gleichen Radius aufweisen. Somit ist sichergestellt, dass eine einzige Knochenschraube im gesamten Langloch 22 verschiebbar ist.

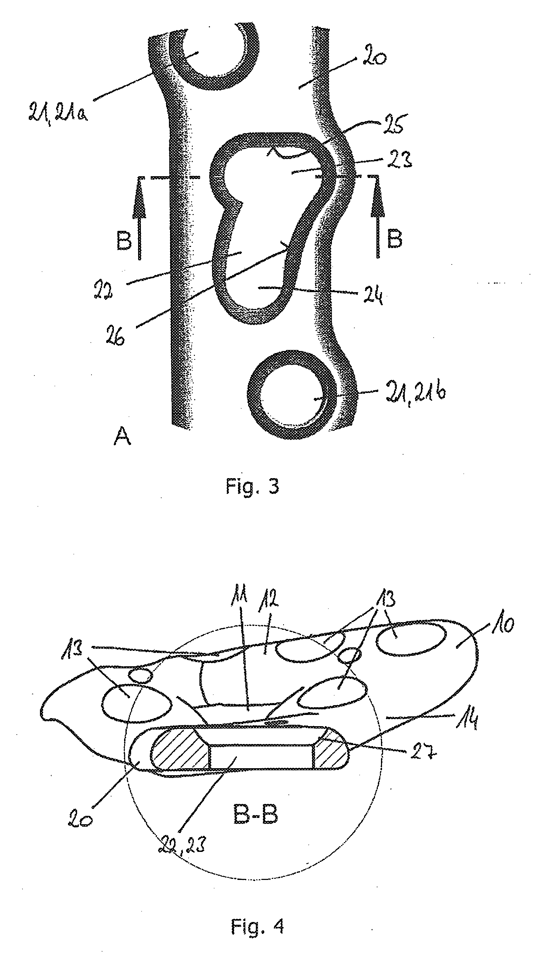

In der Detailansicht gemäß Figur 3, die das Detail A aus Figur 1 zeigt, ist gut zu erkennen, dass der zweite Schenkel 24 einen gekrümmten Verlauf aufweist. Der erste Schenkel 23 weist hingegen einen geraden Verlauf auf. Konkret weist der erste Schenkel 23 eine Längsseite 25 auf, die sich im Wesentlichen geradlinig und rechtwinklig zur Plattenlängsrichtung erstreckt. Der zweite Schenkel 24 weist hingegen eine Längsseite 26 auf, die eine Krümmung aufweist, wobei sich die Längsseite 26 in Richtung des ersten Schenkels 23 krümmt.

Aus Figur 3 ist auch die Anordnung der ersten Bohrung 21a und der zweiten Bohrung 21b in Relation zum Langloch 22 gut erkennbar. Insbesondere sind die erste Bohrung 21a und die zweite Bohrung 21b derart angeordnet, dass eine gedachte Verbindungslinie zwischen den Mittelpunkten der ersten Bohrung 21a und der zweiten Bohrung 21b das Langloch 22, insbesondere den zweiten, schräg verlaufenden Schenkel 24, kreuzt. Um einen ausreichenden diagonalen Versatz zwischen der ersten Bohrung 21a und der zweiten Bohrung 21b zu erreichen, sind die Seitenkanten des Schaftteils 20 im Bereich der ersten Bohrung 21a und der zweiten Bohrung 21b ausgewölbt. Ebenfalls ist die Seitenkante des Schaftteils 20 im Bereich des ersten Schenkels 23, insbesondere im Übergangsbereich vom ersten Schenkel 23 zum zweiten Schenkel 24 des Langlochs 22 ausgewölbt, so dass ein erster Schenkel 23 mit einer erhöhten Länge realisierbar ist.

Im Allgemeinen ist der erste Schenkel 23 kürzer als der zweite Schenkel 24 des Langlochs 22 ausgebildet. Umgekehrt ist der zweite Schenkel 24 länger als der erste Schenkel 23 ausgebildet. Auf diese Weise wird die Längserstreckung des Schaftteils 20 vorteilhaft genutzt, um einen längeren Verschiebeweg innerhalb des zweiten Schenkels 24 für eine Knochenschraube bereitzustellen. Eine verbesserte und präzisere Ausrichtung des Schaftteils 20 bzw. allgemein der Osteosyntheseplatte wird dadurch erreicht.

In Figur 4 ist ein Querschnitt entlang der Linie B-B aus Figur 3 dargestellt. Figur 4 zeigt somit die Osteosyntheseplatte gemäß Figur 1, die entlang der Längsachse des ersten Schenkels 23 geschnitten ist. Darin ist zu erkennen, dass das Langloch 22 eine Senkung 27 aufweist. Die Senkung 27 erstreckt sich über etwa die Hälfte des Querschnitts des Langlochs 22. Dies reicht aus, um den Kopf einer Knochenschraube vollständig aufzunehmen. Ein Überstand einer Knochenschraube über die Oberfläche der Osteosyntheseplatte wird somit vermieden. Im Allgemeinen können auch zumindest ein Teil der Bohrungen 21 im Schaftteil 20 sowie ein Teil der Bohrungen 13 im Kopfteil 10 jeweils eine Senkung 27 aufweisen. Vorzugsweise weisen alle Bohrungen 13 zur Aufnahme von Knochenschrauben eine Senkung 27 auf.

In der Draufsicht auf die Osteosyntheseplatte gemäß Figur 1 ist ferner zu erkennen, dass im proximalen Abschnitt des Schaftteils 20 ein weiteres Langloch 28 angeordnet ist, das sich geradlinig in Plattenlängsrichtung erstreckt. Das weitere Langloch 28 ist zur Aufnahme einer Knochenschraube angepasst und umfasst ebenfalls eine Senkung 27, die zur Aufnahme des Schraubenkopfes der Knochenschraube dient.

Weiter proximal sind im Schaftteil 20 zwei Bohrungen 21 angeordnet, wobei eine dritte Bohrung 21c mit dem weiteren Langloch 28 fluchtet. Das bedeutet, dass die dritte Bohrung 21c in Plattenlängsrichtung mit dem weiteren Langloch 28 ausgerichtet ist. Eine vierte Bohrung 21d ist bezogen zur Längsachse des weiteren Langlochs 28 versetzt angeordnet. Insbesondere weisen die dritte Bohrung 21c und die vierte Bohrung 21d eine gedachte Verbindungslinie auf, die sich schräg zur Plattenlängsrichtung erstreckt.

Der Schaftteil 20 weist zwischen der zweiten Bohrung 21b und der vierten Bohrung 21d mehrere Verjüngungen auf, in denen die Breite des Schaftteils 20 reduziert ist. Insbesondere ist jeweils zwischen zwei Bohrungen 21 bzw. zwischen einer Bohrung 21 und dem weiteren Langloch 28 eine Verjüngung angeordnet. Dies trägt zur Materialeinsparung und Gewichtsreduzierung bei.

Die Erfindung eignet sich insbesondere zur Stabilisierung von Knochenfrakturen des distalen Radius bzw. der distalen Ulna. Dazu wird der Knochen, falls erforderlich, zunächst reponiert, um die ursprünglich physiologische Knochenstellung einzustellen. Anschließend wird die erfindungsgemäße Osteosyntheseplatte derart auf dem Knochen ausgerichtet, dass der Kopfteil 10 im Bereich der Epiphyse und der Schaftteil 20 im Bereich der Diaphyse angeordnet sind. Der Kopfteil 10 wird durch Knochenschrauben, die durch die Bohrungen 13 im Kopfteil geführt werden, an der Epiphyse fixiert. Eine weitere Knochenschraube wird durch das Langloch 22 in die Diaphyse bzw. den Knochenschaft eingebracht. Bevor der Schaftteil 20 vollständig fixiert wird, kann die Osteosyntheseplatte ausgerichtet werden, wobei das Langloch 22 mit der in den Knochenschaft eingedrehten Knochenschraube als Führung dient. Sobald die Osteosyntheseplatte ausgerichtet ist, wird der Schaftteil 20 durch weitere Knochenschrauben, die durch die Bohrungen 21 des Schaftteils 20 und das weitere Langloch 28 geführt werden, fixiert.

Im Allgemeinen können wenigstens ein Teil der Bohrungen 13, 21 im Kopfteil 10 bzw. im Schaftteil 20 ein Innengewinde aufweisen. Insbesondere können alle Bohrungen 13, 21 der Osteosyntheseplatte ein Innengewinde umfassen. Auf diese Weise werden auf die Knochenschrauben wirkende Zugkräfte bzw. Spannungen reduziert, so dass ein Ausreißen von Knochenschrauben aus dem Knochengewebe vermieden wird.

### Bezugszeichenliste

- 10: Kopfteil
- 11: Durchbruch
- 12: Steg
- 13: Bohrung im Kopfteil 10
- 14: Kröpfung
- 20: Schaftteil
- 21: Bohrung im Schaftteil 20
- 21a: erste Bohrung
- 21b: zweite Bohrung
- 21c: dritte Bohrung
- 21d: vierte Bohrung
- 22: Langloch
- 23: erster Schenkel
- 24: zweiter Schenkel
- 25: Längsseite des ersten Schenkels 23
- 26: Längsseite des zweiten Schenkels 24
- 27: Senkung
- 28: weiteres Langloch

## Patentansprüche

1. Osteosyntheseplatte, insbesondere Radiusplatte oder Ulnarplatte, zur Stabilisierung von Knochenfrakturen umfassend einen Kopfteil (10) zur Anordnung an einem Knochenendstück und einen Schaftteil (20) zur Anordnung an einem Knochenschaft, wobei der Kopfteil (10) und der Schaftteil (20) jeweils Bohrungen (21) zur Aufnahme von Knochenschrauben aufweisen, wobei der Schaftteil (20) wenigstens ein Langloch zur Aufnahme einer Knochenschraube aufweist, das zumindest abschnittsweise schräg zur Plattenlängsrichtung, insbesondere zur Schafteillängsrichtung, angeordnet ist,
**dadurch gekennzeichnet, dass**
das Langloch (22) einen ersten Schenkel (23) und einen zweiten Schenkel (24) aufweist, wobei der erste Schenkel (23) im Wesentlichen quer, insbesondere rechtwinklig, zur Plattenlängsrichtung, insbesondere zur Schaftteilslängsrichtung, und der zweite Schenkel (24) im Wesentlichen schräg, insbesondere geneigt, zur Plattenlängsrichtung, insbesondere zur Schafteillängsrichtung, angeordnet ist, wobei in einem proximalen Abschnitt des Schaftteils (20) ein weiteres Langloch (28) angeordnet ist, welches sich geradlinig in Plattenlängsrichtung erstreckt.

2. Osteosyntheseplatte nach Anspruch 1,
**dadurch gekennzeichnet, dass**
das Langloch (22) hakenförmig, insbesondere in Form einer 7, ausgebildet ist.

3. Osteosyntheseplatte nach Anspruch 2,
**dadurch gekennzeichnet, dass**
der erste Schenkel (23) kürzer als der zweite Schenkel (24) ist.

4. Osteosyntheseplatte nach Anspruch 2 oder 3,
**dadurch gekennzeichnet, dass**
der zweite Schenkel (24) einen gekrümmten Verlauf aufweist.

5. Osteosyntheseplatte nach einem der Ansprüche 1-4,
**dadurch gekennzeichnet, dass**
der Schaftteil (20) zwei Bohrungen (21a, 21b) zur Aufnahme von Knochenschrauben aufweist, deren Verbindungslinie schräg zur Plattenlängsrichtung, insbesondere zur Schaftteillängsrichtung, verläuft und das Langloch (22), insbesondere den zweiten Schenkel (24) des Langlochs, kreuzt.

6. Osteosyntheseplatte nach einem der Ansprüche 1-5,
**dadurch gekennzeichnet, dass**
das Langloch (22) eine Senkung (27) aufweist.

7. Osteosyntheseplatte nach einem der Ansprüche 1-6,
**dadurch gekennzeichnet, dass**
zwischen dem Kopfteil (10) und dem Schaftteil (20) eine Kröpfung (14) angeordnet ist.

8. Osteosyntheseplatte nach einem der Ansprüche 1-7,
**dadurch gekennzeichnet, dass**
der Schaftteil (20) bezogen auf die Plattenlängsrichtung eine Länge und eine Breite aufweist, wobei die Länge größer als die Breite ist.

9. Osteosyntheseplatte nach Anspruch 8,
**dadurch gekennzeichnet, dass**
der Kopfteil (10) bezogen auf die Plattenlängsrichtung eine Breite aufweist, die größer als die Breite des Schaftteils (20) ist.

## Claims

1. An osteosynthesis plate, particularly a radius plate or ulnar plate, for stabilizing bones fractures comprising a headpiece (10) to be arranged at the end of a bone and a shank (20) to be arranged on a bone shaft, wherein both the headpiece (10) and the shank (20) comprise bore holes (21) for receiving bone screws, wherein the shank (20) comprises at least one elongated hole for receiving a bone screw, at least a portion of which is arranged diagonal to the longitudinal direction of the plate, particularly the longitudinal direction of the shank,
**characterized in that**
the elongated hole (22) comprises a first flank (23) and a second flank (24), wherein the first flank (23) is disposed substantially transverse, particularly perpendicular, to the longitudinal direction of the plate, particularly the longitudinal direction of the shank, and the second flank (24) is disposed substantially diagonal, particularly oblique, to the longitudinal direction of the plate, particularly the longitudinal direction of the shank, wherein a further elongated hole (28) extending lineally in the longitudinal direction of the plate is arranged in a proximal section of the shank (20).

2. The osteosynthesis plate according to claim 1,
**characterized in that**
the elongated hole (22) is of hook-like design, particularly in the form of a 7.

3. The osteosynthesis plate according to claim 2,
**characterized in that**
the first shank (23) is shorter than the second shank (24).

4. The osteosynthesis plate according to claim 2 or 3,
**characterized in that**
the second shank (24) is curvilinear.

5. The osteosynthesis plate according to any one of claims 1 to 4,
**characterized in that**
the shank (20) comprises two bore holes (21a, 21b) for receiving bone screws, their connecting line running diagonal to the longitudinal direction of the plate, particularly the longitudinal direction of the shank, and crossing the elongated hole (22), particularly the second flank (24) of the elongated hole.

6. The osteosynthesis plate according to any one of claims 1 to 5,
**characterized in that**
the elongated hole (22) comprises a countersink (27).

7. The osteosynthesis plate according to any one of claims 1 to 6,
**characterized in that**
a bend (14) is arranged between the headpiece (10) and the shank (20).

8. The osteosynthesis plate according to any one of claims 1 to 7,
**characterized in that**
the shank (20) has a length and a width relative to the longitudinal direction of the plate, wherein the length is greater than the width.

9. The osteosynthesis plate according to claim 8,
**characterized in that**
the headpiece (10) has a width relative to the longitudinal direction of the plate which is greater than the width of the shank (20).

## Revendications

1. Plaque d'ostéosynthèse, en particulier plaque radiale ou plaque de Ulnar, pour la stabilisation de fractures osseuses, comprenant une partie de tête (10) destinée à être agencée sur un morceau terminal d'un os et une partie de tige (20) destinée à être agencée sur une tige osseuse, dans laquelle la partie de tête (10) et la partie de tige (20) comportent chacune des perçages (21) pour recevoir des vis à os, dans laquelle la partie de tige (20) comporte au moins un trou oblong pour recevoir une vis à os, qui est agencé au moins par portion en oblique par rapport à la direction longitudinale de la plaque, en particulier par rapport à la direction longitudinale de la partie de tige,
**caractérisée en ce que**
le trou oblong (22) comporte une première branche (23) et une seconde branche (24), telles que la première branche (23) est agencée sensiblement transversalement et en particulier à angle droit par rapport à la direction longitudinale de la plaque, en particulier par rapport à la direction longitudinale de la partie de tige, et la seconde branche (24) est agencée sensiblement en oblique et en particulier de façon inclinée, par rapport à la direction longitudinale de la plaque, en particulier par rapport à la direction longitudinale de la partie de tige, dans laquelle un autre trou oblong (28), qui s'étend en ligne droite dans la direction longitudinale de la plaque, est agencé dans un tronçon proximal de la partie de tige (20).

2. Plaque d'ostéosynthèse selon la revendication 1,
**caractérisée en ce que** le trou oblong (22) est réalisé en forme de crochet, en particulier sous la forme d'un 7.

3. Plaque d'ostéosynthèse selon la revendication 2,
**caractérisée en ce que** la première branche (23) est plus courte que la seconde branche (24).

4. Plaque d'ostéosynthèse selon la revendication 2 ou 3, **caractérisée en ce que** la seconde branche (24) présente un tracé incurvé.

5. Plaque d'ostéosynthèse selon l'une des revendications 1 à 4,
**caractérisée en ce que** la partie de tige (20) comporte deux perçages (21a, 21 b) pour recevoir des vis à os, dont la ligne de liaison s'étend en oblique par rapport à la direction longitudinale de la plaque, en particulier par rapport à la direction longitudinale de la partie de tige, et croise le trou oblong (22), en particulier la seconde branche (24) du trou oblong.

6. Plaque d'ostéosynthèse selon l'une des revendications 1 à 5,
**caractérisée en ce que** le trou oblong (22) comporte un renfoncement (27).

7. Plaque d'ostéosynthèse selon l'une des revendications 1 à 6,
**caractérisée en ce qu'**un coude (14) est agencé entre la partie de tête (10) et la partie de tige (20).

8. Plaque d'ostéosynthèse selon l'une des revendications 1 à 7,
**caractérisée en ce que**, par référence à la direction longitudinale de la plaque, la partie de tige (20) présente une longueur et une largeur telles que la longueur est supérieure à la largeur.

9. Plaque d'ostéosynthèse selon la revendication 8,
**caractérisée en ce que**, par référence à la direction longitudinale de la plaque, la partie de tête (10) présente une largeur qui est supérieure à la largeur de la partie de tige (20).
